Europäisches Patentamt

European Patent Office

Office européen des brevets

(11) Publication number: **0 203 084 B1**

## EUROPEAN PATENT SPECIFICATION

(45) Date of publication of patent specification: **12.08.92** (51) Int. Cl.⁵: **B01D 61/00**

(21) Application number: **85904934.8**

(22) Date of filing: **26.09.85**

(86) International application number:
**PCT/US85/01841**

(87) International publication number:
**WO 86/02858 (22.05.86 86/11)**

(54) ADAPTIVE FILTER CONCENTRATE FLOW CONTROL SYSTEM AND METHOD.

(30) Priority: **15.11.84 US 671576**

(43) Date of publication of application:
**03.12.86 Bulletin 86/49**

(45) Publication of the grant of the patent:
**12.08.92 Bulletin 92/33**

(84) Designated Contracting States:
**AT BE CH DE FR GB LI NL SE**

(56) References cited:
**WO-A-82/03567**
**US-A- 4 370 983**

(73) Proprietor: **BAXTER INTERNATIONAL INC. (a Delaware corporation)**
**One Baxter Parkway**
**Deerfield Illinois 60015(US)**

(72) Inventor: **PRINCE, Paul, Richard**
**15960 Maidstone Street**
**Fountain Valley, CA 92708(US)**
Inventor: **FORD, Michael, Gregory**
**3375 Santa Cruz**
**Riverside, CA 92507(US)**
Inventor: **SCHOENDORFER, Donald, Walter**
**1842 Whitestone Terrace**
**Santa Ana, CA 92705(US)**
Inventor: **CLARK, Ronald, Leigh**
**15042 Anita Circle**
**Westminster, CA 92683(US)**

(74) Representative: **Bass, John Henton et al**
**REDDIE & GROSE 16 Theobalds Road**
**London WC1X 8PL(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### 1. Field of the Invention

This invention relates to the optimization of concentrate flow in a filter apparatus and more particularly to a system using actual sensed operating curve data to optimize plasma flow in a plasmapheresis system.

### 2. Discussion of the Prior art

Conventional filtering systems involve a tripartite fluid flow relative to a porous membrane. Feed fluid which is to be filtered is presented to a first side of the membrane and flows across the first side of the membrane along a longitudinal axis of the filter system. Filtrate fluid passes through the membrane and is withdrawn from the second side of the membrane, opposite the first side. Components of the feed fluid which pass along the membrane without passing there through are drawn off as a retentate or concentrate fluid.

In a typical filtering application such as a plasmapheresis system wherein plasma is separated from whole blood to form a packed cell concentrate, it is desirable to maximize the percentage or absolute flow rate of the filtrate. For moderate filtrate flow rates and fixed membrane area, the filtrate flow rate is approximately proportional to transmembrane pressure (TMP).

However, as the filtrate flow rate increases a reversible blocking effect causes transmembrane pressure to increase more rapidly relative to filtrate flow. The blocking effect is reversible in the sense that if the filtrate flow is decreased after blocking has occurred there is no plugging of the filter pores and the original substantially linear TMP-filtrate flow relationship is reestablished.

However, if the filtrate flow (and TMP) become sufficiently high, red cells, platelets or other particulate matter lodge permanently in the membrane pores and begin to irreversibly plug the filter. The plugging decreases the effective area of the membrane and if continued over a period of time will cause the filtrate flow rate to decrease while the TMP remains constant or even increases, or cause an increase in TMP if the filtrate flow rate is maintained. If the filtrate flow rate is decreased the filter membrane remains partially plugged and the effective area of the membrane is permanently decreased wherein the original TMP-filtrate flow relationship is changed undesirably.

One filter flow control system is partially described in Lysaght, M.J.; Schmidt, B.; Samtleen, W.; and Gurland, H.J. "Transport Considerations in Flat Sheet Microporous Membrane Plasmapheresis," Plasma Therapy Transfusion Technology, Vol. 4, No. 4 (1983) pp. 373-85. The described system uses a pumping system with pumps driving the feed fluid (blood) and filtrate (plasma). The control system senses pump flow rates in the feed fluid and filtrate paths and senses pressure in all three filter fluid paths. A clamp also controls the flow of feed fluid over the membrane surface. The sensed information is used by an undisclosed control algorithm to control the filtrate flow and clamp to keep TMP constant and to maintain a desired inlet to outlet pressure differential.

Some systems use a capillary separator in place of a flat membrane device. The capillary separator uses hollow fibers with thin, porous walls. The walls of the fibers are essentially porous membranes and function in a manner similar to a flat membrane. A system using a capillary separator is described in Buchholz, D.H.; Porten, J.; Anderson M.; Helphigstine, C.; Lin, A.; Smith, J.; Path, M.; McCullough, J,; and Snyder, E.; "Plasma Separation Using the Fenwal COS-10 Capillary Plasma Separator," Plasmapheresis, edited by Y. Nose, P.S. Malcesky, J.W. Smith and R.S. Krakauer, Raven Press, New York (1983).

### Summary of the Invention

An adaptive filter fluid flow control system in accordance with the invention includes a filter apparatus having a porous membrane filter, flow regulating having pumps coupled to drive feed fluid, concentrate and filtrate through the filter system, a pressure sensor coupled to detect and indicate transmembrane pressure and a data processor coupled to control the pumping system to optimize filtrate flow rates in response to transmembrane pressure (TMP).

The data processor detects data for at least one actual system TMP-filtrate flow rate operating point, extrapolates the actual operating point data to form a prediction curve and translates the prediction curve to derive a control curve. The control curve may be optimally rotated relative to the prediction curve to obtain specialized control characteristics.

The filter apparatus is operated at a point where actual system operating characteristics represented by a fluid characteristics curve intersect the control curve. The value by which the control curve is translated relative to the prediction curve is selected to place the operating point at an optimum filtration flow rate at which reversible blocking of the filter device has begun to occur. At this point further increases in filtrate flow would soon damage the filter while a lesser flow would result in the harvesting of less filtrate.

Thus, rather than simply monitoring and maintaining a constant TMP, the apparatus of the present invention monitors and controls both TMP and feed fluid, concentrate and filtrate flow rates to maintain the filtrate flow rate at the maximum level consistent with avoidance of irreversible blocking of the filter membrane.

The present invention has particular advantage when used in a plasmapheresis system wherein the feed fluid is whole blood, the concentrate is packed cells and the filtrate is plasma. In a plasmapheresis system the blood flow rate is controlled to match requirements of the donor subject and the plasma flow rate is controlled relative to the blood flow rate. Because the sum of the concentrate and filtrate flow rates must match the feed flow rate, plasma flow rate can be controlled by actively controlling the blood and packed cell flow rates by controlling the speed of peristaltic pumps which pump these fluids.

Brief Description of the Drawings

A better understanding of the invention may be had from a consideration of the following Detailed Description taken in conjunction with the accompanying drawings in which:

Fig. 1 is a schematic and block diagram representation of an adaptive filter fluid flow control system in accordance with the invention;

Fig. 2 is a graphical illustration of curves that are useful in understanding the operation of the filter system shown in Fig. 1;

Fig. 3 is a flow chart illustrating a control sequence for the flow control system shown in Fig. 1;

Fig. 4 is a detailed block diagram and schematic representation of a repetitive motor control update cycle shown generally in Fig. 3; and

Fig. 5 is a detailed block diagram representation of a portion of the motor control update cycle shown in Fig. 4.

Detailed Description

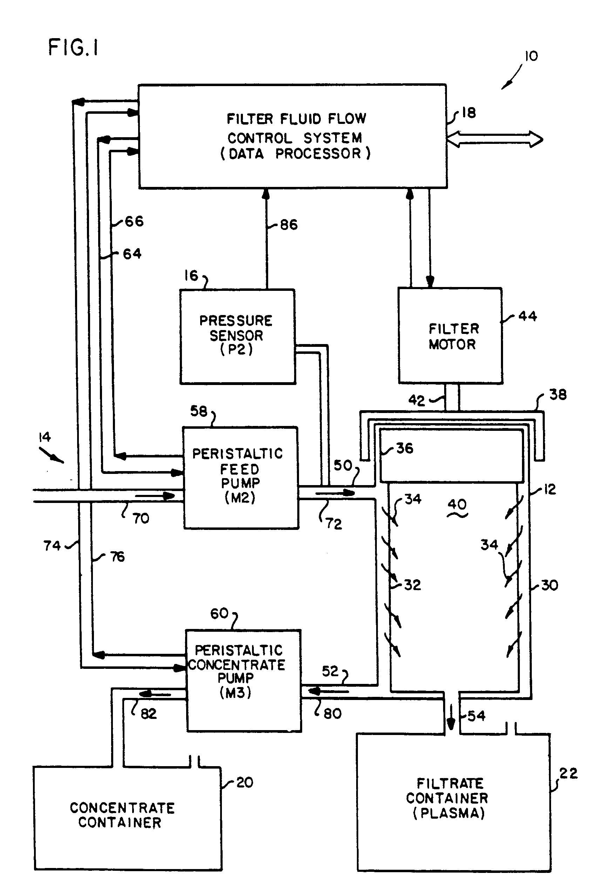

Referring now to Fig. 1, there is shown an adaptive filter fluid flow control system 10 in accordance with the invention including a filter apparatus 12, a pumping system 14, a P2 pressure sensor 16, data processor 18, a concentrate container 20 and a filtrate container 22. While the adaptive filter fluid flow control system 10 may be utilized for other appropriate filtering applications, it is particularly useful for filtering plasma from whole blood in a plasmapheresis system. While the disclosure of this patent has been limited in scope to the subject adaptive filter fluid flow control system 10, a more complete disclosure of a plasmapheresis system in which such an adaptive filter fluid flow control system 10 might be utilised is found in a co-pending, commonly assigned patent application Serial No. 06/626,034 filed June 29, 1984 by Paul Prince et al for "Blood Extraction and Reinfusion Flow Control System and Method".

The filter apparatus 12 may alternatively be a stationery flat membrane or capillary separator type of system but is preferably of the vortex enhanced shear flow separator type having a generally cylindrical housing 30 enclosing a cylindrically shaped rotating filter membrane 32 having pores through which a filtrate such as blood plasma may flow as indicated by arrows 34.

Two magnetic pole pieces 36, 38 provide a magnetic coupling through the hermetically sealed filter housing 30 to cause a rotator 40 supporting the filter membrane 32 and magnetic coupling 36 to rotate in response to a filter motor 44 and shaft 42. Filter motor 44 is conventionally driven at a controlled velocity in response to commands from the data processor 18. Filter motor 44 includes position feedback sensing devices in the form of Hall effects device which return to data processor 18 twelve uniformly distributed pulses for each rotation of filter motor 44.

The present invention is not concerned with the specific configuration of the filter apparatus 12. The filter apparatus 12 has thus been represented in a somewhat schematic and idealized form. However, a more complete description of a preferred configuration for the filter apparatus 12 can be found in a commonly assigned patent application Serial No. 591,925 filed March 21, 1984 for "Method and Apparatus for Separation of Matter from Suspension", by Donald W. Schoendorfer.

In operation, the filter apparatus 12 receives an anticoagulant protected whole blood feed fluid at an inlet 50. The feed fluid surrounds the cylindrical, rotating membrane 32 and flows longitudinally downward toward a concentrate or retentate outlet 52 through which the concentrate or retentate is discharged which is packed cells for the presently disclosed application of use in a plasmapheresis system. As the feed fluid flows longitudinally downward from the inlet 50 to the outlet 52 a filtrate component thereof, which is plasma in the present instance, passes through pores in the filter membrane 32 as indicated by arrows 34 to exit the filter apparatus 12 through a filtrate outlet 54 which is coupled to the filtrate container 22. The filtrate container 22 is schematically illustrated as being vented to the atmosphere. However, in the preferred embodiment the container 22 is actually a flexible wall bottle or container which maintains the filtrate fluid at atmospheric pressure while isolating the fluid from atmospheric contamination.

The pumping system 14 includes a peristaltic feed pump 58 and a peristaltic concentrate pump 60. The peristaltic feed pump 58 includes a drive motor designated M2 which is coupled in a conventional manner to receive velocity commands from filter fluid flow control system 18 over a control path 64. Like the filter motor 44 the motor M2 driving peristaltic feed pump 58 includes conventional Hall effect position sensor devices which provide to data processor 18 over a feedback path 66 rotational position indicating pulses with 12 pulses distributed over each cycle of rotation of the motor M2. Peristaltic feed pump 58 receives feed fluid through a conduit 70 from a suitable feed fluid source such as a donor subject in the particularly disclosed application and pumps the received feed fluid with a controlled flow velocity to the inlet 50 of filter apparatus 12 through a conduit 72.

The peristaltic concentrate pump 60 is coupled in a conventional manner to receive velocity control signals over a control path 74 from data processor 18. Peristaltic concentrate pump 60 includes a drive motor M3 which receives the velocity control commands and has Hall effect position feedback sensing devices. The Hall effect devices provide to data processor 18 position feedback signals in the form of 12 pulses uniformly distributed over each rotation of the motor M3. The feedback pulses are communicated to the data processor 18 over a feedback path 76.

Peristaltic concentrate pump 60 receives retentate fluid from outlet 52 over a conduit 80 and pumps the retentate fluid at a controlled flow rate over a conduit 82 to the concentrate container 20. As with the filtrate container 22, the concentrate container 20 is shown having a vent to atmospheric pressure. However, it should be kept in mind that in the present application the concentrate within concentrate container 20 must be protected from atmospheric contamination as by using a filtration type of vent.

It can readily be seen that once the filter apparatus 12 is primed with fluid, the flow of feed fluid through inlet 50 must equal the flow of concentrate fluid through outlet 52 plus the flow of filtrate fluid through outlet 54. Consequently, by controlling the flow of concentrate fluid through peristaltic concentrate pump 60 relative to the flow of feed fluid through peristaltic feed pump 58, the flow of filtrate fluid through outlet 54 is inherently controlled even though no pump is disposed along the filtrate fluid path to directly control the flow of filtrate fluid. It is thus apparent that references to control of the filtrate fluid made in this disclosure can include indirect control over filtrate fluid flow by controlling the feed fluid flow and concentrate fluid flow as well as direct control over filtrate fluid flow.

It is further noted that in general any relative control of the fluid rates through feedpump 58 and concentrate pump 60 can be utilized to control the filtrate fluid flow rate, their differences equalling the filtrate flow rate for incompressible fluids. However, for the present application of a plasmapheresis system, it has been found desirable to control the feedpump 58 independently of the filter apparatus 12 to optimize the extraction of feed fluid from a subject donor serving as the source of feed fluid. The concentrate pump 60 is then controlled relative to the flow rate through the feedpump 58 to produce a system controlled filtrate flow rate through outlet 54. Alternatively, pump 60 could be positioned to control filtrate fluid flow through outlet 54 directly . The remaining discussion assumes that pump 60 is positioned along the concentrate fluid path between conduits 80 and 82.

P2 pressure sensor 16 is coupled to sense pressure along the conduit 72 between the peristaltic feedpump 58 and the inlet 50 of filter apparatus 12. Pressure sensor 16 responds to the sensed pressure by generating indications thereof which are communicated over a pressure feedback path 86 to the data processor 12. Because pressure sensor 16 is coupled to a path which is in direct flow relationship with an inlet side of the membrane 32 and because the filtrate which is in direct flow relationship with an outlet side of the membrane 32 is maintained at a constant, atmospheric pressure, the pressure sensed by sensor 16 is indicative of transmembrane pressure across the membrane 32, subject to corrections for pressure loss in the conduit 72 between the sensor point and the inlet 50, subject to pressure head that is dependent upon the particular relative elevations of the conduit 72 and the filtrate container 22, subject to centrifugally induced pressures resulting from rotation of the rotor 40, and subject to hydrodynamic pressure loss through the filter device 30.

The structure of the data processor 18 is not disclosed in detail. However, it may be implemented as a conventional microprocessor based control system having conventional analog to digital convertors and digital to analog convertors coupling the microprocessor to the motors and feedback devices of the adaptive filter system 10. Concentrate container 20 temporarily stores concentrate until full, wherein the plasmapheresis system pauses in the operation of filter 30 in order to return the concentrate to the fluid feed source. A combination of one filtration episode and one concentrate return comprises a filtration cycle.

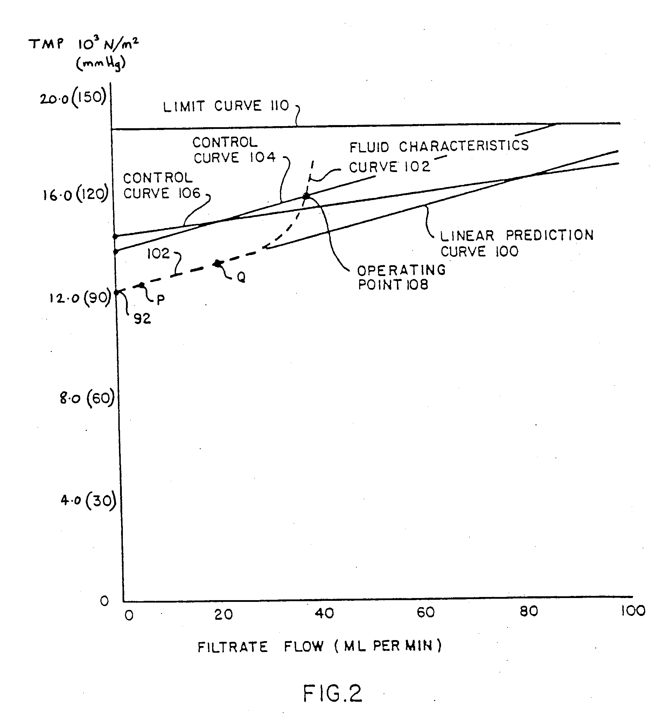

Curves illustrating the method of controlling filtrate flow through a filter system in accordance with the present invention are shown in Fig. 2 to which reference is now made. In the present example each filtration operation or session begins with an initializing interval during which the feedpump 58 is operated at a constant and relatively low feed fluid flow rate of 50 milliliters per minute. This flow rate is selected to be well within the supply rate capacity for the feed fluid. During this initializing interval the filter apparatus 12 is primed with fluid and once stable operation is obtained, initializing measurements are taken. These measurements include the sensing and storing of pressure P2 at one or more relatively low filtrate flow rates. For example, pressure data may be acquired at a flow rate of 5 milliliters per minute as indicated at a point P and at 20 milliliters per minute as indicated at a point Q. Typical operating characteristics would result in a sensed pressure of $12.5x10^3N/m^2$ (94mmHg) above atmospheric at point P and a pressure of $13.3x10^3N/m^2$(100mmHg) above atmospheric at point Q.

The sensed initializing points are then fitted with a prediction curve which in the case of two sensed points is a straight line forming a linear prediction curve 100. The linear prediction curve 100 will typically have a slope of $1.1x10^3N/m^2$ (8mmHg) per 20ml per minute concentrate flow rate and intersect the 0 flow rate axis at $12.3x10^3N/m^2$(92mmHg).

This $12.3x10^3N/m^2$ intersection point 92 results from flow rate independent constant pressure offsets occurring at the pressure sensing point near the inlet 50 of filter apparatus 12. While this pressure offset will vary significantly with the particular implementation and use of the filter apparatus 12, when used in a plasmapheresis system, the offset might typically be comprised of $12.7x10^3N/m^2$ (95mmHg) from centrifugal force induced pressure, $-4.7x10^3N/m^2$(-35mmHg) from the pressure head resulting from elevational differences between the pressure sensor and the filtrate container 22, and $4.3x10^3N/m^2$(32mmHg)from convective turbulence forces within the filter apparatus 12.

The exact transmembrane pressure is thus the pressure sensed at P2 less these fixed offsets less some additional minor corrections for flow related pressure drops such as drops within conduit 72. However, because these flow rate related pressure changes tend to vary linearly with flow rate as sensed at pressure sensor P2 they will vary linearly at the pressure sensor P2 and can be automatically included in the linear prediction curve 100. The slope of the linear prediction curve 100 thus can be considered to represent changes in actual TMP, although a small percentage of those changes are actually induced by other sources.

At lower plasma flow rates, the actual sensed fluid pressure will follow the linear prediction curve 100. However, as the filtrate flow rate increases (for a constant feed flow rate), the filter membrane or other filter device will begin to experience reversible blocking and actual sensed pressure increases more rapidly than the linear prediction curve 100 as represented by a fluid characteristic curve 102.

Translation of the linear prediction curve 100 upward by an experimentally determined pressure offset of $1.6x10^3N/m^2$(12mmHg) results in a control curve 104 which intersects the fluid characteristic curve 102 at an operating point 108.

At the operating point 108, filtrate flow is maximized or optimized at a point above the linear prediction curve 100 where reversible blocking has begun to occur but irreversible plugging of the membrane pores is not taking place. If the pressure offset translation magnitude between the linear prediction curve 100 and control curve 104 is made too great, the operating point 108 will occur at a point having too high a TMP and irreversible plugging of the filter membrane 32 will begin to occur. As this plugging proceeds over time the effective membrane area will decrease from the initial area of 40 square centimeters and the slope of the fluid characteristics curve 102 will increase. That is, curve 102 will tend to be translated upward and rotated counter clockwise as the effective filter area is reduced. Hence, the operating point 108 will begin to move toward lower plasma flow rates and down the control curve 104 toward the 0 flow rate axis. However, so long as the offset is chosen to enable the control curve 104 to intersect the fluid characteristic curve 102 at an operating point 108 below the pressure and filtrate flow rate at which irreversible plugging begins to occur, stable operation of the filter apparatus 12 may be obtained at a relatively high filtrate flow rate, maximized by the measurement of the pressure-flow characteristics of the instant filter device and the instant feed fluid characteristics.

Since the control curve 104 is mathematically determined in response to the actual sensed operating point Q, or points P and Q, the slope of the control curve can be varied relative to the linear prediction curve 100 to improve operating conditions which may depend upon the particular application and filtering configuration of the filter apparatus 12. For example, rotation of the control curve about the 20 milliliter per minute flow rate intercept point from a nominal slope of $1.1x10^3N/m^2$(8mmHg)per 20 milliliter per minute flow rate to $0.5x10^3N/m^2$(4mmHg) per 20 milliliter per minute flow rate results in a control curve 106. Control curve 106 produces an operating point intercept with fluid characteristics curve 102 slightly below the operating point 108 but its reduced slope also produces a greater differential with respect to the fluid characteristics curve 102 at lower filtrate flow rates. The greater differential can be utilized to cause an error driven flow control system controlling the filtrate flow rate to more rapidly increase the filtrate flow rate toward the eventual relatively high operating point. The lower slope control curve 106 also tends to more severely limit the TMP at the operating point as filtrate flow rate increases. Greater protection against irreversible plugging is thus provided for the filter membrane at higher filtrate flow rates. It will be noted of course that less protection is accordingly afforded at lower filtrate flow rates. It will be appreciated that depending upon the particular fluid characteristics and filter system characteristics other adaptive control curve shapes might be utilized to optimize operation of the adaptive control system 10.

A limit curve 110 imposes an upper limit of $18.7 \times 10^3 N/m^2 (140 mmHg)$ on the allowable operating point transmembrane pressure as sensed at P2 pressure sensor 16. Should the detected intersection of the fluid characteristic curve 102 with the control curve 104 indicate an operating point 108 with a TMP greater than 140, the lesser value of 140 is imposed as an operating point. The limit point of $18.7 \times 10^3 N/m^2 (140 mmHg)$ is selected to be above the anticipated operating point so as to avoid interference with expected normal system operation, yet sufficiently low to protect the filter membrane against rapid plugging should an error condition or unexpected operating condition occur, such as a significant error in the measurement of point P or Q, or unusual variation in the viscosity characteristics of the feed fluid.

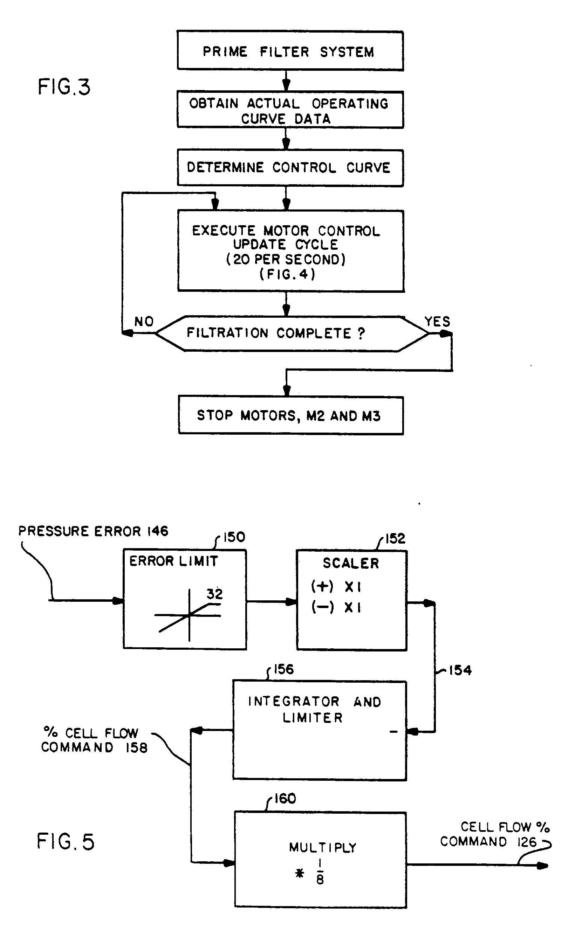

The operating sequence of the data processor 18 is illustrated in Fig. 3 to which reference is now made. As noted previously, the data processor 18 performs all data processing activities for the plasmapheresis system of which the adaptive filter flow control system 10 is a portion. The data processor 18 controls the velocity of all motors in the plasmapheresis system including filter motor 44, motor M2 for peristaltic feed pump 58, and motor M3 for peristaltic concentrate pump 60. Normal processor operations are interrupted every 50 milliseconds to update motor velocity commands. During a typical motor control update cycle the processor detects and stores all of the feedback data, including positional and time information from the motors 44, 58 and 60 and pressure information over line 86 from P2 pressure sensor 16. The processor then updates the velocity commands for the filter motor 44, motor M2 for peristaltic feed pump 58 and then motor M3 for peristaltic concentrate pump 60.

While in general the peristaltic feed pump 58 could be utilized to control the rate of concentrate flow from filter apparatus 12, in the present application it is desired to control the peristaltic feed pump 58 in response to an availability of feed fluid and adapt the pumping rate of peristaltic concentrate pump 60 to changes in the feed fluid flow rate to maintain a desired concentrate flow rate. Other implementations of the concepts of this invention involve the use of a filtrate pump wherein the filtrate is commanded In the present example, optimal control is thus obtained by first updating the M2 motor velocity command for peristaltic feed pump 58 so that current velocity information is available during the course of the same update cycle when motor velocity commands for motor M3 driving peristaltic concentrate pump 60 are updated.

Operation for a given filtration operation or session begins with priming of the filter apparatus 12 and the conduit tubing connecting the filter apparatus 12 to the pumps 58, 60 and to filtrate container 22. This priming operation is conducted at relatively low, constant flow rates which are experimentally predetermined to be well within the flow capabilities of the system. For example, the peristaltic feed pump 58 may be operated at a flow rate of 50 milliliters per minute while the peristaltic concentrate pump 60 is operated at a flow rate of 30 milliliters per minute which accordingly results in a concentrate flow rate of 20 milliliters per minute (40% of the feed fluid flow rate) through filter apparatus 12 and into container 22. The priming operation stabilizes system operating conditions and particularly the pressure head which becomes a constant and contributes to the 0 flow rate axis intercept of the linear prediction curve 100 (Fig. 2).

After priming has stabilized system operating points, the next executed step is to obtain actual operating curve data. During this operating step actual operating point data is sensed such as the pressure point Q, or two pressure points P and Q for filtrate flow rates of 5 and 20 milliliters per minute respectively as shown in Fig. 2. Typical sensed pressure might be $12.5 \times 10^3 N/m^2 (94 mmHg)$ at 5 milliliters per minute and $13.3 \times 10^3 N/m^2 (100 mmHg)$ at 20 milliliters per minute. When the prediction curve 100 is linearly extrapolated from these two points the linear prediction curve 100 has a slope of (Pressure Q-Pressure P)$N/m^2$ /(20-5) ml/minute. In some cases this slope may be substantially consistent from donation to donation. In this case only a single point, such as point P or Q may be required, using a predetermined slope of $1.7 \times 10^3 N/m^2/20ml/min$ for example.

In practice it has been found advantageous to use as a sensed actual operating point the average of a plurality of different data samples. The averaging of a plurality of samples tends to smooth out the substantial pressure pulses which are induced by the peristaltic feedpump 58 and to compensate for a relatively low resolution of the P2 pressure sensor 16. While higher quality pressure sensors and data converters are of course available, for the present application it has been found that the best pressure sensor and data converter which is economically suitable has a resolution of only $0.5 \times 10^3 N/m^2 (4 mmHg)$ in the least significant bit. By using as the final pressure value at a given operating point, an average of 4 or 5 or more samples, the effective resolution of the P2 pressure sensor 16 can be increased to about $0.2 \times 10^3 N/m^2$ (1.5mmHg).

After actual operating point data has been obtained at 1 or more points such as points P and Q in Fig. 2, the data processor 18 determines the control curve 104. As indicated previously, the control curve 104 may be generated by simply translating the linear prediction curve 100 upward by a fixed pressure offset value such as $1.6 \times 10^3 N/m^2$ (12mmHg) as in the present example. Alternative derivations may be utilized as well, depending upon the particular application.

It is desirable to use points P and Q to calculate the prediction curve 100 to accurately reflect the actual characteristics of the feed fluid, the pump flow constants, and the filter configuration. Since the curve slope and thus the extrapolated curve is generated from two relative pump command flow rates, scale errors between pump command flow rates and actual flow rates are eliminated if two or more calibration points are used.

However, in the present example it has been found to be economically expedient to use only a single actual operating point which has been selected to be point Q. Experiments have shown that for the specific application of filtering plasma from whole blood, the slope of the linear prediction curve 100 remains fairly uniform at approximately $1.1 \times 10^3 N/m^2$(8mmHg) per 20ml per minute flow rate from session to session not withstanding substantial changes in the hematocrit dependent viscosity of the whole blood feed fluid. At the same time, the resolution of the P2 pressure sensor 16 is relatively coarse compared to the $1.1 \times 10^3 N/m^2$ per 20mililiters per minute flow rate slope of the linear prediction 100. As a result of these factors, it has been found advantageous for the specific application of a plasmapheresis system to use an experimentally predetermined slope of $1.1 \times 10^3 N/m^2$per 20 milliliter per minute flow rate for the linear prediction curve 100 and position it at the pressure point Q actually sensed at the filtrate flow rate of 20 milliliters per minute. The control curve 104 is then determined as having a $1.6 \times 10^3 N/m^2$ (12mmHg) offset from this linear prediction curve 100 having a predetermined slope and a vertical position (pressure offset value) determined from actual sensed operating point data.

Once the control curve 104 is established the system proceeds to execute motor control update cycles 20 times per second until the filtration process is complete. When filtration is complete the motor M3 driving concentrate pump 60 is stopped along with other motors in the plasmapheresis system.

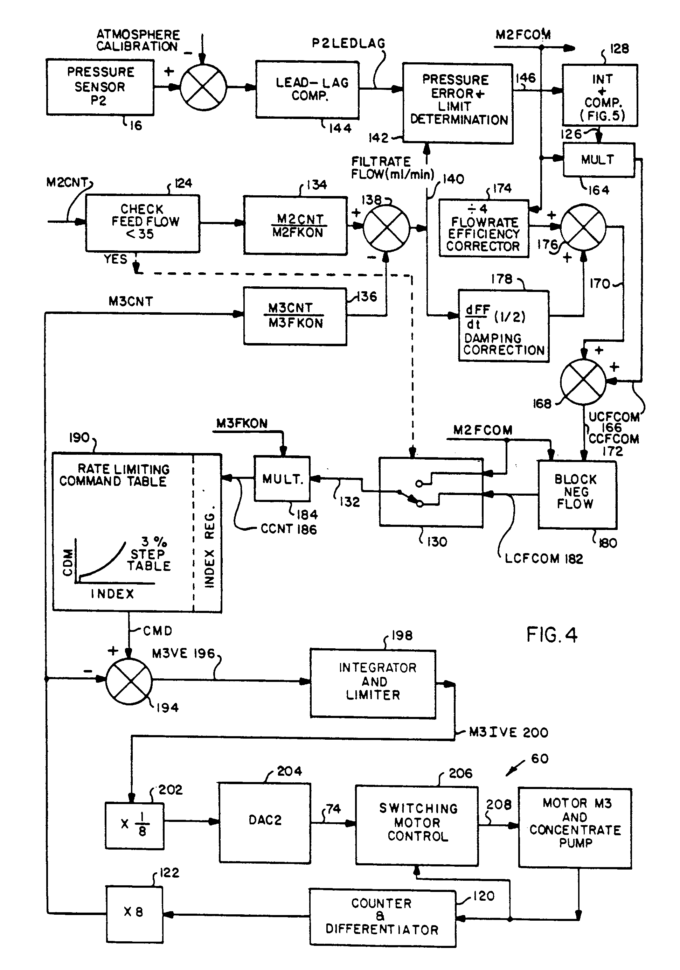

The servo control used in updating the velocity of the motor M3 driving the concentrate pump 60 is illustrated in greater detail in Fig. 4, to which reference is now made. It should be appreciated that while Fig. 4 illustrates the velocity update cycle for motor M3 in block diagram and schematic form, the mathematical and conditional operations represented by the various blocks are actually executed by the data processor 18.

As indicated previously, the velocity control update cycle begins with sensing of all system conditions and calculation of actual feedback motor velocities. The updating of motor velocity for motor M3 is typical of the procedure for all motors in the system and is executed at a counter and differentiator step 120. Employed by this step is a hardware counter which is incremented by each feedback pulse from motor M3. This counter is thus a positional reference indicating 12 times the number of revolutions of motor M3. This position count is in effect differentiated to produce a velocity signal by subtracting the position count for the previous update cycle from the present position count. The current position count is then stored for use in the next update cycle. The current velocity count is then scaled by multiplying it by eight at a step 122 to produce a motor M3 count signal M3CNT. In similar fashion a motor 2 count signal M2CNT is determined for the motor M2 for feedpump 58.

At step 124 the velocity signal M2CNT is checked to see if the motor M2 has reached a velocity corresponding to a feed flow rate of 35 milliliters per minute. If yes, normal operation is assumed and a full update cycle is executed. However, if the detected feed flow velocity is less than 35 milliliters per minute, a special case (such as at startup) or other abnormal operating condition is presumed and the concentrate pump 60 is constrained to match the flow rate of the feed fluid pump 58 so that a filtrate flow rate of 0 is produced. This is accomplished by arbitrarily setting a concentrate or cell flow percent command 126 output from an integrator and comparator step 128 to a value representing 100%. As will be apparent from a more detailed description below, this corresponds to a filtrate or plasma flow rate of 0 and precludes undesirable transients in the servo loop once the feed fluid or whole blood flow rate exceeds 35 milliliters per minute and the filtrate flow rate is then permitted to ramp up from 0 to a steady state flow rate. As the integrator and comparator is set to output the 100% value a virtual switch 130 is set to produce as M3 velocity command output 132 the previously updated feedpump motor M2 flow command, M2FCOM. This effectively commands the motor M3 to follow the velocity of motor M2.

Under normal circumstances the feed fluid flow will be 35 milliliters per minute or greater and the system then proceeds to divide signal M2CNT by a flow constant M2FKON at a step 134 and divide signal M3CNT by a motor 3 flow constant M3FKON at a step 136. These division steps 134, 136 merely provide a unit conversion from the raw motor count units to a flow rate of milliliters per minute utilized in the servo control loop. A subtractor 138 subtracts the concentrate flow rate from the feed fluid flow rate to produce a filtrate fluid flow rate signal 140 at the output thereof. This filtrate flow signal 140 becomes one input to a pressure error and limit determination step 142. Pressure error and limit determination step 142 also receives as a second input a compensated pressure signal, P2LEDLAG, which is obtained by receiving a current actual pressure indication from P2 pressure sensor 16, calibrating the pressure signal by subtracting actual atmospheric pressure, and imposing lead lag compensation upon the calibrated pressure signal at a step 144.

Within pressure error and limit determination step 142 the filtrate flow signal 140 is utilized to access the control curve 104 (Fig. 2). The value on control curve 104 at the indicated filtrate flow rate is calculated and if this value is greater than the limit curve 110 value of $18.7 \times 10^3 N/m^2$ (140 mmHg), the smaller value of $18.7 \times 10^3 N/m^2$ will be used for the control curve 104 pressure value. Normally, the actual control curve 104 pressure value will be the smaller and the compensated actual pressure value represented by signal P2LEDLAG is subtracted from this value to produce a pressure error signal 146. The pressure error signal 146 is integrated and compensated at a step 128 which is shown in greater detail in Fig. 5 to which reference is now made.

Referring now to Fig. 5, the pressure error signal 146 is subjected to an error limit step 150 at which a positive upper limit of $4.3 \times 10^3 N/m^2$ (32mmHg) is placed upon the pressure error signal 146. The limited pressure error signal is then subject to a scaler step 52 at which separate paths are available to multiply positive or negative pressure error signals by a constant. In the present application the constant has a value of "one" for both positive and negative values but the mechanism is made available to permit different scaling for positive and negative values where a particular application might make separate scaling desirable. The scaler 152 can conceptually be thought of as providing a change of units of measurement and outputs a signal 154 which is effectively a percent concentrate flow error signal.

Integrator and limiter 156 receives the percent cell flow error signal 154, performs a sign change, integrates the error signal to produce a percent cell flow command signal 158 and imposes upon the percent cell flow command signal 158 an upper limit equal to a constant P2RSHLIM and lower limit equal to a constant P2RSLLIM. In the present application the upper limit P2RSHLIM has a value of 2047 corresponding to a packed cell or concentrate flow rate of 100 percent of the feed fluid flow or 0 percent filtrate flow rate. Similarly, the lower limit has a value of +266 which corresponds to a concentrate flow rate of approximately 37 percent of the feed fluid flow rate or a filtrate flow rate of a approximately 63 percent of the feed fluid flow rate. This lower limit on the percent cell flow command 158 in effect operates as a safety feature preventing excess filtrate flow through a filter.

Referring back to Fig. 4, the cell flow percent command 126 is communicated to a multiplier 164 which multiplies the cell flow percent command 126 times the commanded feed flow motor velocity command M2FCOM and simultaneously performs a scaling function by dividing the product by 256. Output of multiplier 164 is thus an uncompensated concentrate flow command 166 commanding concentrate flow in units of milliliters per minute.

The uncompensated flow command 166 is communicated to an adder 168 where it is added to a compensation signal 170 to produce a compensated cell flow command, CCFCOM 172.

A filtrate flow rate efficiency corrector 174 receives the commanded motor 2 velocity signal, M2FCOM, divides it by 4, and presents the result to an adder 176. The efficiency correction dynamically feeds forward a correction to substantially compensate any known filter efficiency variations as a function flow rate by adjusting the cell flow rate. For example, at a feed fluid flow rate of 60 ml per min a typical stabilized operating point might permit a yield of 80% of available plasma. If the available plasma is 60% of the feed flow rate, then the plasma or filtrate flow rate will be 48% of the feed fluid flow rate or 28.8 ml per minute. As the feed fluid flow rate increases to 100ml per min the operating point may stabilize with filtrate being only 70% of the available flow rate. This would reduce the stabilized filtrate flow rate to 42.0 ml per min.

However, if the control system follows a rapid change in feed fluid flow rate from 60 to 100 ml per min with a constant percentage filtrate flow rate, the instantaneous filtrate flow rate will be 48.0 ml per minute. This will produce a pressure error signal which will gradually reduce the filtrate flow rate to the stable 42.0 ml per min operating point. However, in the mean time the filter 32 may be experiencing irreversible plugging. The feed forward of the feed fluid command signal M2FCOM through flow rate efficiency corrector 174 enables the motor control system to more quickly adjust to changes in feed fluid flow rates and thus reduce or eliminate periods during which the filter 32 experiences reversible blocking. A differentiating step 178 receives the actual measured filtrate flow signal 140 which is presented to the pressure error limit determination step 142, differentiates this signal 140 by subtracting from the current value thereof the value obtained in the previous update cycle, divides the difference by 2, and presents half the difference to adder 176. Adder 176 adds 1/2 the derivative of the filtrate flow rate to 1/4 the feed fluid motor command signal to produce a compensation signal 170.

The compensated flow command 172 is communicated to a block negative flow step 180 where checks are made to assure that neither concentrate fluid or filtrate fluid have a negative flow. Negative flow of concentrate fluid is blocked by limiting the compensated concentrate flow command 172 to a value greater than or equal to 0. Negative filtrate flow is prevented by limiting the compensated concentrate flow command signal 172 to values less than or equal to the feed flow motor velocity command signal, M2FCOM. The block negative flow step 180 outputs a limited concentrate flow command signal 182 to the virtual switch step 130. It will be recalled that step 130 typically applies the limited concentrate flow command signal 182 to the multiplier 132. However, in the event that feed flow is less than a predetermined minimum of 35 milliliters per minute, virtual switch 130 substitutes the feed motor flow command M2FCOM for the M3 velocity command signal 132 which is applied to the multiplier 184.

Multiplier 184 performs a scaling function to convert from milliliters per minute to flow velocity in terms of Hall device feedback counts per update cycle. Multiplier 184 multiplies the M3 velocity command signal by a motor flow constant M3FKON which relates the output concentrate count command signal 186 to the M3 velocity command 132.

The concentrate count command 186 is presented to an exponential rate limiting command table 190. Rate limiting command table 190 imposes a rate of increase limit upon the change of fluid flow velocity produced by the peristaltic concentrate pump 60. In effect, the concentrate flow rate is permitted to increase at a rate of only 3% per update interval but may be rapidly decelerated. Although not specifically shown, the feed flow peristaltic pump 58 is similarly limited to flow acceleration rates of 3% per update time interval. Consequently, during transient acceleration conditions, the concentrate flow is maintained as a substantially constant percent of feed flow as the two pumps 58, 60 are being accelerated, both limited to 3% change per update cycle.

The rate limiting command table is implemented as a stored look up table storing the command values which form the output motor 3 commanded count signal, M3CCNT. These values are addressed by an index value stored in an index register within rate limiting command table 190 (physically within a data store age location in filter fluid flow control system 18). At an index value of 0 the stored command value is 0 to assure a commanded 0 velocity for the peristaltic concentrate pump 60. At an index value of 1 the command value, CMD, has an experimentally predetermined value selected to overcome system offsets and friction forces to provide the minimum value which will sustain rotation of motor M3 driving peristaltic concentrate pump 60. Thereafter, the command value, CMD, is increased by three percent for each unity increment in the index address value.

As a further limit upon the acceleration of the peristaltic concentrate pump 60, the value accessed by the current index is compared to the concentrate count signal 186 during each update cycle. If the concentrate count signal 186 is less than the value accessed by the current index, the command value, CMD, is set to the concentrate count signal 1CCNT 186. On the other hand, if the concentrate count signal 186 is greater than the stored table value accessed by the current index, the index value is incremented by 1. After a sufficient number of update cycles a steady state condition will be reached wherein the index value stored in the index register accesses a tabulated command value equal to or slightly greater than the concentrate count signal 186.

The actual M3 motor count signal, M3CNT is subtracted from the velocity command signal, M3CCNT, at a subtracter step 194 to generate a motor M3 velocity error signal, M3VE 196. The velocity error signal 196 is then integrated at an integrator and limiter step 198 to produce an integrated velocity error signal, M3IVE 200. Integrator and limiter 198 imposes an upper limit representing 100 milliliters per minute upon the integrated velocity error signal, M3IVE 200.

Integrated velocity error signal 200 is then scaled by multiplication by 1/8 at a multiplier 202 and then communicated to a digital-to-analog converter 204 designated DAC2. Digital-to-analog converter 204 is an actual hardware device which is represented as being contained within the data processor 18 in Fig. 1. The analog output from DAC2 204 is communicated to a switching motor control system 206 over line 74. Switching motor control system 206 is represented as being part of peristaltic concentrate pump 60 in Fig. 1 and converts the analog velocity command received over line 74 to a switched energization pulse 208 which actually energizes motor M3 which in turn rotates the concentrate pump.

Referring again to Fig. 3, the motor control update cycle is periodically executed 20 times per second until a filtration subcycle has been completed. After completion of the filtration subcycle the feed fluid motor M2 and the concentrate fluid motor M3 are both commanded to stop and execution of the update cycle is terminated.

**Claims**

1. A method of optimising a flow of filtrate through a membrane filter having feed thereto and concentrate and filtrate flows therefrom characterised by the steps of:

priming the filter by operating at a relatively low constant fluid flow rate to establish a non-obstructed filter pore flow of concentrate through the filter;

determining at least one actual non-obstructed filter pore actual filter operating point (P) representing the functional relation between filtrate flow rate and filter pressure, the said operating point (P) being determined by detecting the transmembrane pressure at the said relatively low constant fluid flow rate;

generating a flow control curve (104 or 106) having a shape conforming to a sensed non-obstructed transmembrane pressure curve located by said operating point (P) and an empirically determined curve slope, and having a predetermined pressure offset and rotation relative to said operating point (P) and said pressure offset; and

controlling the rate of filtrate flow so that, in operation, the sensed actual filter pressure is that at an operating point (108) lying on the flow control curve (104).

2. A method according to claim 1, further characterised in that the step of generating the flow control curve (104) includes generating a prediction curve (100) passing through the detected at least one non-obstructed filter pore actual filter operating point (P).

3. A method according to claim 2, further characterised by the predication curve (100) being a straight line on a linear co-ordinate system.

4. A method according to claim 2, further characterised by the detecting step detecting exactly one non-obstructed filter pore actual filter operating point (P) and the prediction curve (100) being linear and having a predetermined slope.

5. A method according to claim 2, further characterised by the detecting step detecting exactly two non-obstructed filter pore actual filter operating points (P,Q) and by the prediction curve (100) being linear and passing through the two detected non-obstructed filter pore actual filter operating points (P,Q).

6. A method according to claim 1, further characterised by the steps of determining a plurality of actual non-obstructed filter pore transmembrane pressure-filtrate flow rate operating points dependent upon actual feed fluid, fluid pumping system, and filter membrane characteristics encountered during each operation of the filter and determining the particular control curve in response to the determined actual operating points.

7. A method according to claim 6, further characterised by the steps of causing the feed fluid, concentrate fluid and filtrate fluid to flow at controlled flow rates in response to at least one control parameter; and using a three dimensional pressure verses feed fluid flow rate verses filtrate flow rate coordinate system to determine at least one filter system actual operating point and to determine from the at least one actual operating point a control curve having a pressure offset relative to a locus of anticipated non-obstructed filter pore actual operating points; and generating the at least one control parameter to control the flow rates in response to a pressure difference between an actual sensed pressure and a pressure at a corresponding point on the control curve to tend to reduce the pressure difference, these relationships being scaled linearly with feed fluid flow rates.

8. A method according to claim 7, including the steps of integrating the pressure difference and generating the at least one control parameter to control the flow rates in response to the integral of the pressure difference.

9. A method according to claim 8, further characterised by the at least one control parameter dictating the feed fluid velocity and further controlling the flow rates to tend to reduce the pressure difference in response to a product of the integral of the pressure difference and the dictated feed fluid velocity.

10. A method according to claim 9, further characterised by the at least one control parameter representing a flow rate efficiency correction to further maximise the safe operating filtrate flow rate.

11. A flow control system for controlling a flow of filtrate through a filter apparatus (12) including a filter membrane (32) with a predictable transmembrane pressure-filtrate flow rate relationship when the filter apparatus is operated under non-obstructed filter pore operating conditions, characterised by:

a flow regulating system (14) coupled to control flows of feed fluid, concentrate fluid and filtrate fluid in the filter apparatus in response to at least one control signal;

a pressure sensor (16) disposed to sense and generate indications of transmembrane pressure in the filter apparatus; and

a data processor (18) coupled to receive an indication of transmembrane pressure from the pressure sensor and generate the at least one control signal to control the flow regulating system in response thereto to maintain the transmembrane pressure at an intersection with a particular curve having a shape conforming to the predictable transmembrane pressure-filtrate flow rate relationship and a pressure offset from the received pressure indication by a predetermined pressure difference.

12. A flow control system according to claim 11, further characterised in that the filter membrane (32) has a predictable transmembrane pressure-filtrate flow rate relationship which is a substantially linear relationship in which pressure increases with filtrate flow rate.

13. A flow control system according to claim 11 or claim 12, further characterised by the filter apparatus (12) being coupled to receive and filter a feed fluid, by the filter membrane (32) being a porous membrane which separates filtrate fluid from the feed fluid to produce concentrate fluid, the flow of filtrate fluid through the membrane having the predictable transmembrane pressure filtrate flow rate relationship; and by the flow regulating system (14) including means (58,60) for pumping, the means for pumping being disposed to control the flow of feed fluid, concentrate fluid and filtrate fluid in the filter apparatus.

14. A flow control system according to any of claims 11 to 13, further characterised by the flow regulating system (14) including a concentrate pump (60) coupled to pump concentrate fluid from the filter apparatus (12) and by the at least one control signal from the data processor (18) controlling the pumping system (14) by controlling the flow rate of the concentrate pump.

15. A flow control system according to claim 14, further characterised by the flow regulating system (14) including a feed fluid pump (58) operating to pump feed fluid to the filter apparatus (12) the said control signal controlling the flow rate of the concentrate pump relative to the flow rate of the feed fluid pump.

16. A flow control system according to any of claims 11 to 13, further characterised by the flow regulating system (14) including a feed fluid pump (58) operating to pump feed fluid to the filter apparatus (12) and a filtrate pump coupled to pump filtrate fluid from the filter apparatus and by the at least one control signal from the data processor (18) controlling the pumping system by controlling the flow rate of the filtrate pump relative to the flow rate of the feed fluid pump.

17. A flow control system according to any of claims 11 to 16, further characterised by the predetermined pressure difference being a positive pressure difference.

18. A flow control system according to any of claims 11 to 17, further characterised by the feed fluid being blood, the concentrate being packed cells, and the filtrate being plasma.

19. A flow control system according to any of claims 11 to 18, further characterised by the filter membrane (32) being a rotating membrane and the filtrate having a centripetal flow direction through the rotating membrane.

**Revendications**

1. Procédé d'optimisation du débit d'un filtrat au travers d'un filtre à membrane recevant une alimentation et délivrant un concentré et un filtrat, caractérisé par les étapes de :

amorçage du filtre par mise en fonctionnement à un débit de fluide constant relativement faible afin d'établir un écoulement à pores de filtre non obstrués du concentré au travers du filtre ;

la détermination d'au moins un point de fonctionnement réel de filtre à pores de filtre non obstrués (P) qui représente la relation fonctionnelle entre le débit du filtrat et la pression du filtre, ledit point de fonctionnement (P) étant déterminé en détectant la pression transmembrane audit débit de fluide constant relativement faible ;

la génération d'une courbe de régulation de débit (104 ou 106) qui présente une forme qui se conforme à une courbe de pression transmembrane, dans une condition non obstruée, détectée et positionnée au moyen dudit point de fonctionnement (P) et au moyen d'une pente de courbe déterminée de manière empirique et qui présente un décalage de pression prédéterminé et une rotation relative par rapport audit point de fonctionnement (P) et par rapport audit décalage de pression ; et

la régulation du débit du filtrat de telle sorte qu'en fonctionnement, la pression de filtre réelle détectée soit celle d'un point de fonctionnement (108) qui est placé sur la courbe de régulation de débit (104).

2. Procédé selon la revendication 1, caractérisé en outre en ce que l'étape de génération de la courbe de régulation de débit (104) inclut la génération d'une courbe de prédiction (100) qui passe par l'au moins un point de fonctionnement de filtre réel détecté à pores de filtre non obstrués (P).

3. Procédé selon la revendication 2, caractérisé en outre par la courbe de prédiction (100) qui est une ligne rectiligne dans un système de coordonnées linéaires.

4. Procédé selon la revendication 2, caractérisé en outre par l'étape de détection qui détecte exactement un point de fonctionnement de filtre réel à pores de filtre non obstrués (P) et par la courbe de prédiction (100) qui est linéaire et qui présente une pente prédéterminée.

5. Procédé selon la revendication 2, caractérisé en outre par l'étape de détection qui détecte exactement deux points de fonctionnement de filtre réels à pores de filtre non obstrués (P, Q) et par la courbe de prédiction (100) qui est linéaire et qui passe par les deux points de fonctionnement de filtre réels détectés à pores de filtre non obstrués (P,Q).

6. Procédé selon la revendication 1, caractérisé en outre par les étapes de détermination d'une pluralité de points de fonctionnement réels de pression transmembrane - débit de filtrat à pores de filtre non obstrués qui dépendent du fluide d'alimentation réel, du système de pompage de fluide et des caractéristiques de la membrane de filtre rencontrés lors de chaque fonctionnement du filtre, et de la détermination de la courbe de régulation particulière en réponse aux points de fonctionnement réels déterminés.

7. Procédé selon la revendication 6, caractérisé en outre par les étapes qui consistent à amener le fluide d'alimentation, le fluide concentré et le fluide filtré à s'écouler selon des débits régulés en réponse à au moins un paramètre de régulation ; et à utiliser un système de coordonnées à trois dimensions constitué par la pression en fonction du débit du fluide d'alimentation et du débit du filtrat afin de déterminer au moins un point de fonctionnement réel du système de filtre et afin de déterminer à partir de l'au moins un point de fonctionnement réel une courbe de régulation qui présente un décalage de pression par rapport à un lieu de points de fonctionnement réels anticipés à pores de filtre non obstrués ; et à générer l'au moins un paramètre de régulation pour réguler les débits en réponse à une différence de pression entre une pression réelle captée et une pression en un point correspondant sur la courbe de régulation pour tendre à réduire la différence de pression, ces relations étant mises à l'échelle de manière linéaire avec des débits de fluide d'alimentation.

8. Procédé selon la revendication 7, incluant les étapes d'intégration de la différence de pression et de génération de l'au moins un paramètre de régulation pour réguler les débits en réponse à l'intégrale de la différence de pression.

9. Procédé selon la revendication 8, caractérisé en outre par l'au moins un paramètre de régulation qui dicte la vitesse du fluide d'alimentation et qui régule en outre les débits pour tendre à réduire la différence de pression en réponse à un produit de l'intégrale de la différence de pression et de la vitesse de fluide d'alimentation dictée.

10. Procédé selon la revendication 9, caractérisé en outre par l'au moins un paramètre de régulation qui représente une correction de rendement de débit pour maximiser encore le débit du filtrat en fonctionnement sûr.

11. Système de régulation de débit pour commander un débit de filtrat au travers d'un appareil de filtrage (12) incluant une membrane de filtre (32) avec une relation pression transmembrane-débit de filtrat qui peut être prédite lorsque l'appareil de filtrage est actionné dans des conditions de fonctionnement à pores de filtre non obstrués, caractérisé par :

un système de régulation de débit (14) couplé à des débits de régulation de fluide d'alimentation, de fluide concentré et de fluide filtré contenus dans l'appareil de filtrage en réponse à l'au moins un signal de régulation ;

un capteur de pression (16) disposé pour capter et générer des indications de pression transmembrane dans l'appareil de filtrage ; et

un processeur de données (18) couplé pour recevoir une indication de pression transmembrane en provenance du capteur de pression et pour générer l'au moins un signal de régulation afin de réguler le système de régulation de débit en réponse à ce signal pour maintenir la pression transmembrane à une intersection avec une courbe particulière qui présente une forme qui se conforme à la relation pression transmembrane-débit de filtrat qui peut être prédite et un décalage de pression par rapport à l'indication de pression reçue au moyen d'une différence de pression prédéterminée.

12. Système de régulation de débit selon la revendication 11, caractérisé en outre en ce que la membrane de filtre (32) présente une relation ou pression transmembrane-débit de filtrat qui peut être prédite et qui est une relation sensiblement linéaire dans laquelle la pression augmente avec le débit du filtrat.

13. Système de régulation de débit selon la revendication 11 ou 12, caractérisé en outre par l'appareil de filtrage (12) qui est couplé pour recevoir et filtrer un fluide d'alimentation, par la membrane de filtre (32) qui est une membrane poreuse qui sépare le fluide filtré du fluide d'alimentation pour produire un fluide concentré, le débit du fluide filtré au travers de la membrane présentant la relation pression transmembrane-débit de filtrat qui peut être prédite ; et par le système de régulation de débit (14) qui inclut un moyen (58, 60) de pompage, le moyen de pompage étant disposé pour réguler le débit du fluide d'alimentation, du fluide concentré et du fluide filtré dans l'appareil de filtrage.

14. Système de régulation de débit selon l'une quelconque des revendications 11 à 13, caractérisé en outre par le système de régulation de débit (14) qui inclut une pompe de concentré (60) couplée pour pomper un fluide concentré depuis l'appareil de filtrage (12) et par l'au moins un signal de régulation qui provient du processeur de données (18) qui régule le système de pompage (14) en régulant le débit de la pompe de concentré.

15. Système de régulation de débit selon la revendication 14, caractérisé en outre par le système de régulation de débit (14) qui inclut une pompe de fluide d'alimentation (58) qui fonctionne pour pomper un fluide d'alimentation jusqu'à l'appareil de filtrage (12), ledit signal de régulation régulant le débit de la pompe de concentré en fonction du débit de la pompe de fluide d'alimentation.

16. Système de régulation de débit selon l'une quelconque des revendications 11 à 13, caractérisé en outre par le système de régulation de débit (14) qui inclut une pompe de fluide d'alimentation (58) qui fonctionne pour pomper du fluide d'alimentation jusqu'à l'appareil de filtrage (12), par une pompe de filtrat couplée pour pomper un fluide filtré depuis l'appareil de filtrage et par l'au moins un signal de régulation qui provient du processeur de données (18) qui régule le système de pompage en régulant le débit de la pompe de filtrat en fonction du débit de la pompe de fluide d'alimentation.

17. Système de régulation de débit selon l'une quelconque des revendications 11 à 16, caractérisé en outre par la différence de pression prédéterminée qui est une différence de pression positive.

18. Système de régulation de débit selon l'une quelconque des revendications 11 à 17, caractérisé en outre par le fluide d'alimentation qui est constitué par du sang, par le concentré qui est constitué par des cellules concentrées et par le filtrat qui est constitué par du plasma.

19. Système de régulation de débit selon l'une quelconque des revendications 11 à 18, caractérisé en outre par la membrane de filtre (32) qui est une membrane tournante et par le filtrat qui présente une direction d'écoulement centripète au travers de la membrane tournante.

**Patentansprüche**

1. Verfahren zum Optimieren eines Filtratdurchflusses durch einen Membranfilter, dem ein Einsatzgut zugeführt wird und aus dem Konzentrat- und Filtratströme austreten, gekennzeichnet durch folgende Schritte:

Vorbereiten des Filters durch Betreiben mit einer relativ geringen konstanten Fluiddurchflußmenge, um einen nichtblockierten Filterporendurchfluß von Konzentrat durch den Filter auszubilden;

Bestimmen von wenigstens einem tatsächlichen Filterarbeitspunkt (P) bei nichtblockierten Filterporen, der die funktionelle Beziehung zwischen Filtratdurchflußmenge und Filterdruck darstellt, wobei der genannte Arbeitspunkt (P) durch Erfassen des Transmembrandrucks bei der genannten relativ geringen konstanten Fluiddurchflußmenge bestimmt wird;

Erzeugen einer Durchflußregelkurve (104 oder 106) mit einem Verlauf, der mit einer erfaßten blockierungsfreien Transmembrandruckkurve, die durch den Arbeitspunkt (P) örtlich festgelegt ist, und einer empirisch bestimmten Kurvenneigung übereinstimmt, und mit einer vorbestimmten Druckabweichung und Drehung relativ zu dem Arbeitspunkt (P) und der Druckabweichung; und

Regeln der Filtratdurchflußmenge derart, daß im Betrieb der erfaßte tatsächliche Filterdruck der Druck an einem Arbeitspunkt (108) ist, der auf der Durchflußregelkurve (104) liegt.

2. Verfahren nach Anspruch 1, ferner dadurch gekennzeichnet, daß der Schritt des Erzeugens der Durchflußregelkurve (104) das Erzeugen einer Vorhersagekurve (100) umfaßt, die durch den erfaßten wenigsten einen tatsächlichen Filterarbeitspunkt (P) bei nichtblockierten Filterporen geht.

3. Verfahren nach Anspruch 2, ferner dadurch gekennzeichnet, daß die Vorhersagekurve (100) eine Gerade auf einem linearen Koordinatensystem ist.

4. Verfahren nach Anspruch 2, ferner dadurch gekennzeichnet, daß im Schritt des Erfassens genau ein tatsächlicher Filterarbeitspunkt (P) bei nichtblockierten Filterporen erfaßt wird, und daß die Vorhersagekurve (100) linear ist und eine vorbestimmte Neigung hat.

5. Verfahren nach Anspruch 2, ferner dadurch gekennzeichnet, daß im Schritt des Erfassens genau zwei tatsächliche Filterarbeitspunkte (P, Q) bei nichtblockierten Filterporen erfaßt werden, und daß die Vorhersagekurve (100) linear ist und durch zwei erfaßte tatsächliche Filterarbeitspunkte (P, Q) bei nichtblockierten Filterporen geht.

6. Verfahren nach Anspruch 1, ferner gekennzeichnet durch folgende Schritte: Bestimmen einer Vielzahl von tatsächlichen Transmembrandruck/Filtratdurchflußmengen-Arbeitspunkten bei nichtblockierten Filterporen in Abhängigkeit von tatsächlichen Charakteristiken von Einsatzfluid, Fluidpumpsystem und Filtermembran, die während jedes Betriebs des Filters auftreten, und Bestimmen der speziellen Regelkurve aufgrund der bestimmten tatsächlichen Arbeitspunkte.

7. Verfahren nach Anspruch 6, ferner gekennzeichnet durch folgende Schritte: Veranlassen des Einsatzfluids, des Konzentratfluids und des Filtratfluids, aufgrund von wenigstens einem Regelparameter mit geregelten Durchflußmengen zu strömen; und Anwenden eines dreidimensionalen Koordinatensystems von Druck zu Einsatzfluiddurchflußmenge zu Filtratdurchflußmenge, um wenigstens einen tatsächlichen Arbeitspunkt des Filtersystems zu bestimmen und um aus dem wenigstens einen tatsächlichen Arbeitspunkt eine Regelkurve mit einer Druckabweichung relativ zu einem Ort von erwarteten tatsächlichen Arbeitspunkten bei nichtblockierten Filterporen zu bestimmen; und Erzeugen des wenigstens einen Regelparameters, um die Durchflußmengen aufgrund einer Druckdifferenz zwischen einem erfaßten tatsächlichen Druck und einem Druck an einem entsprechenden Punkt auf der Regelkurve in Richtung einer Verringerung der Druckdifferenz zu regeln, wobei diese Beziehungen linear mit Einsatzfluiddurchflußmengen skaliert sind.

8. Verfahren nach Anspruch 7, das folgende Schritte umfaßt: Integrieren der Druckdifferenz und Erzeugen des wenigstens einen Regelparameters, um die Durchflußmengen aufgrund des Integrals der Druckdifferenz zu regeln.

9. Verfahren nach Anspruch 8, ferner dadurch gekennzeichnet, daß der wenigstens eine Regelparameter die Einsatzfluidgeschwindigkeit vorschreibt und ferner die Durchflußmengen in Richtung einer Verringerung der Druckdifferenz aufgrund eines Produkts des Integrals der Druckdifferenz und der vorgeschriebenen Einsatzfluidgeschwindigkeit regelt.

10. Verfahren nach Anspruch 9, ferner dadurch gekennzeichnet, daß der wenigstens eine Regelparameter eine Durchflußmengen-Wirkungsgradkorrekturdarstellt, um die sichere Betriebs-Filtratdurchflußmenge zu maximieren.

11. Durchflußsteuersystem zum Steuern eines Filtratdurchflusses durch eine Filtervorrichtung (12), die eine Filtermembran (32) mit einer vorhersagbaren Beziehung von Transmembrandruck zu Filtratdurchflußmenge aufweist, wenn die Filtervorrichtung unter Betriebsbedingungen mit nichtblockierten Filterporen betrieben wird, gekennzeichnet durch:

einen Durchflußregelkreis (14), der gekoppelt ist, um Durchflüsse von Einsatzfluid, Konzentratfluid und Filtratfluid in der Filtervorrichtung aufgrund wenigstens eines Steuersignals zu regeln;

einen Drucksensor (16), der angeordnet ist, um den Transmembrandruck in der Filtervorrichtung zu erfassen und entsprechende Anzeigen zu erzeugen; und

einen Prozessor (18), der gekoppelt ist, um von dem Drucksensor eine Transmembrandruckanzeige zu empfangen und das wenigstens eine Steuersignal zu erzeugen, um den Durchflußregelkreis aufgrund dieses Steuersignals zu steuern, um den Transmembrandruck an einer Schnittstelle mit einer bestimmten Kurve zu halten, deren Verlauf mit der vorhersagbaren Beziehung des Transmembrandrucks zu der Filtratdurchflußmenge und einer Druckabweichung von der empfangenen Druckanzeige um eine vorbestimmte Druckdifferenz übereinstimmt.

12. Durchflußsteuersystem nach Anspruch 11, ferner dadurch gekennzeichnet, daß die Filtermembran (32) eine vorhersagbare Beziehung von Transmembrandruck zu Filtratdurchflußmenge hat, die eine im wesentlichen lineare Beziehung ist, wobei der Druck mit der Filtratdurchflußmenge zunimmt.

13. Durchflußsteuersystem nach Anspruch 11 oder 12, ferner dadurch gekennzeichnet, daß die Filtervorrichtung (12) gekoppelt ist, um ein Einsatzfluid aufzunehmen und zu filtrieren; daß die Filtermembran (32) eine poröse Membran ist, die Filtratfluid von dem Einsatzfluid trennt, um Konzentratfluid zu erzeugen, wobei der Filtratfluiddurchfluß durch die Membran die vorhersagbare Beziehung von Transmembrandruck zu Filtratdurchflußmenge hat; und daß der Durchflußregelkreis (14) eine Einrichtung (58, 60) zum Pumpen aufweist, die angeordnet ist, um den Durchfluß von Einsatzfluid, Konzentratfluid und Filtratfluid in der Filtervorrichtung zu regeln.

14. Durchflußsteuersystem nach einem der Ansprüche 11 bis 13, ferner dadurch gekennzeichnet, daß der Durchflußregelkreis (14) eine Konzentratpumpe (60) aufweist, die gekoppelt ist, um Konzentratfluid aus der Filtervorrichtung (12) zu fördern, und daß das wenigstens eine Steuersignal des Prozessors (18) das Pumpsystem (14) durch Regeln der Durchflußmenge der Konzentratpumpe steuert.

15. Durchflußsteuersystem nach Anspruch 14, ferner dadurch gekennzeichnet, daß der Durchflußregelkreis (14) eine Einsatzfluidpumpe (58) aufweist, die Einsatzfluid zu der Filtervorrichtung (12) fördert, wobei das genannte Steuersignal die Durchflußmenge der Konzentratpumpe relativ zu der Durchflußmenge der Einsatzfluidpumpe steuert.

16. Durchflußsteuersystem nach einem der Ansprüche 11 bis 13, ferner dadurch gekennzeichnet, daß der Durchflußregelkreis (14) eine Einsatzfluidpumpe (58), die Einsatzfluid zu der Filtervorrichtung (12) fördert, und eine Filtratpumpe aufweist, die gekoppelt ist, um Filtratfluid aus der Filtervorrichtung zu fördern, und daß das wenigstens eine Steuersignal des Prozessors (18) das Pumpsystem durch Regeln der Durchflußmenge der Filtratpumpe relativ zu der Durchflußmenge der Einsatzfluidpumpe steuert.

17. Durchflußsteuersystem nach einem der Ansprüche 11 bis 16, ferner dadurch gekennzeichnet, daß die vorbestimmte Druckdifferenz eine positive Druckdifferenz ist.

18. Durchflußsteuersystem nach einem der Ansprüche 11 bis 17, ferner dadurch gekennzeichnet, daß das Einsatzfluid Blut, das Konzentrat gepackte Zellen und das Filtrat Plasma ist.

19. Durchflußsteuersystem nach einem der Ansprüche 11 bis 18, ferner dadurch gekennzeichnet, daß die Filtermembran (32) eine drehende Membran ist und das Filtrat eine zentripetale Durchflußrichtung durch die drehende Membran hat.

FIG.1

FIG.2

FIG.3

```
┌─────────────────────────┐
│   PRIME FILTER SYSTEM    │
└─────────────────────────┘
            │
            ▼
┌─────────────────────────┐
│  OBTAIN  ACTUAL  OPERATING │
│        CURVE DATA         │
└─────────────────────────┘
            │
            ▼
┌─────────────────────────┐
│  DETERMINE CONTROL CURVE │
└─────────────────────────┘
            │
     ┌──────┤
     │      ▼
     │  ┌─────────────────────────┐
     │  │   EXECUTE MOTOR CONTROL  │
     │  │      UPDATE CYCLE        │
     │  │    (20 PER SECOND)       │
     │  │       (FIG. 4)           │
     │  └─────────────────────────┘
     │          │
     │          ▼
  NO │  ◁───────────────────────▷  YES
     └──│  FILTRATION COMPLETE ?  │──┐
        ◁───────────────────────▷   │
                                     │
            ┌────────────────────────┘
            ▼
┌─────────────────────────┐
│  STOP MOTORS, M2 AND M3  │
└─────────────────────────┘
```

PRESSURE ERROR 146

FIG.5

```
                      ┌─150              ┌─152
┌──────────────┐   ┌──────────────┐
│  ERROR LIMIT │   │    SCALER     │
│              │   │   (+)  X I    │
│          32  │   │   (−)  X I    │
│    ╱         │   │               │
└──────────────┘   └──────────────┘
                                   │ ┌─154
              ┌─156                │
        ┌──────────────────┐       │
        │  INTEGRATOR  AND │       │
        │     LIMITER      │       │
% CELL FLOW │              │   −   │
COMMAND 158 │              │◄──────┘
        └──────────────────┘
              ┌─160
        ┌──────────────────┐              CELL FLOW %
        │                  │              COMMAND 126
        │    MULTIPLY      │──────────────►
        │      *  1/8      │
        └──────────────────┘
```

EP 0 203 084 B1

FIG. 4

19